# EUROPÄISCHE PATENTSCHRIFT

(11) **EP 2 421 597 B1**
(45) Veröffentlichungstag und Bekanntmachung des Hinweises auf die Patenterteilung: **01.03.2017**
(21) Anmeldenummer: 10719257.7
(22) Anmeldetag: 22.04.2010
(51) Int. Cl.: A61M 39/20, A61M 39/22

(54) **BRECHVERSCHLUSS ZUM VERSTOPFEN EINES SCHLAUCHS**
FRANGIBLE CLOSURE TO OBSTRUCT A FLEXIBLE TUBE
BOUCHON DE RUPTURE DESTINÉ À BOUCHER UN TUYAU

(30) Priorität: 25.04.2009 DE 102009018979
(43) Veröffentlichungstag der Anmeldung: 29.02.2012
(73) Patentinhaber: Spang & Brands GmbH, 61381 Friedrichsdorf (DE)
(72) Erfinder: MADER, Jürgen, 35516 Dambach (DE)
(74) Vertreter: Patentanwälte Olbricht Buchhold Keulertz
(86) Internationale Anmeldenummer: PCT/EP2010/002473
(87) Internationale Veröffentlichungsnummer: WO 2010/121812

(56) Entgegenhaltungen:
- WO-A1-02/056946
- DE-A1- 4 116 474
- DE-U1- 20 203 154
- DE-U1- 29 706 562
- US-A- 4 915 704

## Beschreibung

Die Erfindung betrifft einen Brechverschluss zum Verstopfen eines an einem Beutel oder Behälter mit einer medizinischen Lösung angeschlossenen Schlauchs bis zur Ingebrauchnahme, bestehend aus einem Brechstopfen mit einem im Querschnitt entsprechend dem inneren Schlauchquerschnitt bemessenen, hohlen, endseitig offenen, unteren Teil, an dessen Ende ein Außengewinde angeformt ist, und einem über eine Sollbruchstelle einstückig mit dem unteren Teil verbundenen, im Querschnitt kleineren, oberen Teil, wobei die Öffnung des unteren Teils durch eine dessen Ende übergreifende, lösbare Verschlusskappe abgedeckt ist.

Ein derartiger Brechverschluss mit einem hohlen, bei Ingebrauchnahme z. B. eines mit eine Natriumchloridlösung gefüllten Beutels durchzubrechenden Brechstopfens mit zugehöriger Verschluss kappe ist z. B. in der DE 41 16 474 C2, der DE 297 06 562 U1 und der WO 02/056 946 A1 beschrieben. Das Durchbrechen des Brechstopfens an der Sollbruchstelle erfolgt von Hand durch Biegen des Schlauchs, nachdem die Verschlusskappe mit Innengewinde von einem passenden Außengewinde auf dem unteren Ende des Brechstopfens abgeschraubt und dieses mit einem Überleitungsgerät, z. B. einem zum Patienten führenden Schlauch mit Adaptern, dicht verbunden worden ist.

Die bekannten Ausführungen derartiger Brechverschlüsse leiden bisher unter mehreren Mängeln. Unbefriedigend ist insbesondere, dass sich infolge ungleichmäßiger Wärmedehnung und Schrumpfung des normalerweise aus Polykarbonat bestehenden Brechstopfens und der z. B. aus Polyoximethylen (POM) bestehenden Verschlusskappe bei dem vorgeschriebenen Sterilisiervorgang die Verbindung zwischen diesen beiden Teilen lockern kann, so dass anschließend Keime in den inneren Hohlraum des Brechstopfens eindringen können. Man geht davon aus, dass bereits nach wenigen Tagen die Sterilität verloren gehen kann. Außerdem lässt sich auch nicht ohne weiteres feststellen, ob die Verschlusskappe schon einige Zeit vor der Ingebrauchnahme der medizinischen Lösung vorübergehend geöffnet und dann wieder auf den Brechstopfen aufgeschraubt worden ist. Aus diesem Grunde besteht ebenfalls Unsicherheit, ob im Zeitpunkt der Ingebrauchnahme tatsächlich noch die notwendige Sterilität gewährleistet ist.

Um die notwendige Sicherheit der Sterilität längerfristig aufrechtzuerhalten, werden deshalb die Behälter mit der medizinischen Lösung mitsamt Auslassschlauch mit Brechverschluss in einen äußeren, dicht verschließbaren Beutel verpackt. Diese Umverpackung erhöht die Kosten und muss erst in umständlicher Weise entfernt werden, bevor die medizinische Lösung angewendet werden kann.

Die DE 202 03 154 U1 zeigt eine alternative Lösung eines Originalitätsnachweises. Hier ist ein Drehverschluss vorgesehen, der zweiteilig mit einem Stützring ausgebildet ist. Beim Lösen des Verschlusses wird die Kappe mit Hilfe von Sollbruchstellen von dem Stützring abgetrennt und der Stützring verbleibt auf dem Brechverschluss. Durch diese Bauweise kann zwar sichergestellt werden, dass die Kappe original verschlossen ist, so lange wie Kappe und Stützring miteinander verbunden sind, allerdings kann ein Drehverschluss keine dauerhafte Sterilität des Brechstopfens gewährleisten.

Während Gegenstand der Erfindung ein Brechverschluss in Form eines mit einer Verschlusskappe abgedeckten Brechstopfens ist, der einen an einen Beutel oder Behälter mit einer medizinischen Lösung angeschlossenen Schlauch dicht verstopft und bestimmungsgemäß nach dem Anschluss dieses Schlauchs an ein mit einem Patienten zu verbindendes Überleitungsgerät durch den Schlauch hindurch aufgebrochen wird, um die Leitungsverbindung zum Patienten zu öffnen, beschreibt die US 4 915 704 A eine an mindestens einem Ende offene Rohrverzweigung, die an einem anderen Ende mittels einer Brechkappe verschlossen bleiben soll, bis es gebraucht wird. Zu diesem Zweck wird die Kappe mit festem Gleitsitz auf dem betreffenden Rohrende montiert. Um sie schnell und einfach abnehmen zu können, ist sie axial außerhalb des Gleitsitzbereichs mit einer Sollbruchstelle geformt, die von einer Schutzkappe aus steiferem Material überdeckt ist, um einem unbeabsichtigten Öffnen der Sollbruchstelle vorzubeugen. Schon zuvor kann jedoch der Innenraum der Rohrverzweigung über ein anderes, offenes Rohrende kontaminiert sein. Außerdem erleichtert die bekannte Brechkappe mit Sollbruchstelle zwar das Öffnen des betreffenden Rohrendes, sorgt aber nicht für eine absolut zuverlässige Abdichtung gegen Keime, da nach wie vor z.B. beim Autoklavieren der feste Gleitsitz der Brechkappe auf dem Rohrende gelockert oder sogar die Brechkappe samt steifer Schutzkappe durch den erhöhten Innendruck abgesprengt werden kann. Schließlich leidet eine solche Brechkappe im Gegensatz zu einem eingangs bezeichneten Brechstopfen unter dem Mangel, dass das verschlossene Rohrende nach dem Aufbrechen der Kappe sofort geöffnet ist, so dass Flüssigkeit austreten kann, bevor ein weiterführender Schlauch angeschlossen worden ist.

Der Erfindung liegt daher die Aufgabe zugrunde, einen Brechverschluss der eingangs genannten Art zur Verfügung zu stellen, dessen innerer Hohlraum nach Herstellung und Sterilisierung auch ohne dichte Umverpackung sehr lange steril bleibt.

Vorstehende Aufgabe wird erfindungsgemäß dadurch gelöst, dass der Innendurchmesser der Verschlusskappe größer ist als der Außendurchmesser des Außengewindes des Brechstopfens und die Verschlusskappe in ihrem oberen Bereich mit dem angeformten Bund stirn- und/oder umfangsseitig durch Kleben oder Schweißen materialschlüssig, ringsum dicht verbunden und dieSollbruchstelle in der Umfangswand axial darunter ausgebildet ist.

Die Erfindung bietet den Vorteil, dass die ringförmige materialschlüssige Verbindung zwischen der Verschlusskappe und dem Brechstopfen eine dicht geschlossene Barriere bildet, die das Eindringen von Keimen zuverlässig und dauerhaft verhindert. Andererseits soll durch diese Sicherheitsmaßnahme nicht die Handhabung beeinträchtigt werden. Daher sieht die Erfindung zusätzlich eine Sollbruchstelle in der Umfangswand der Verschlusskappe vor, die aufreißt, wenn die vorzugsweise mit äußeren axialen Rippen geformte Verschlusskappe in üblicher Weise zum Öffnen gedreht wird. Die Sollbruchstelle hat gleichzeitig die bisher fehlende Funktion einer Originalitätssicherung.

Um das Aufreißen der Sollbruchstelle in der Verschlusskappe zu erleichtern, besteht diese in bevorzugter Ausgestaltung der Erfindung aus einem thermoplastischen Elastomer (TPE) mit einer Härte von vorzugsweise maximal 50 Shore. Infolge der Elastizität des Materials dehnt es sich zu Beginn der Drehbewegung der Verschlusskappe am stärksten im dünnen Querschnittsbereich der Sollbruchstelle. Dabei bilden sich über den Umfang Ungleichmäßigkeiten, und nachdem an einer Stelle ein Anriss entstanden ist, bedarf es auch nur noch eines verhältnismäßig geringen Drehmoments, damit sich die Rissstelle über den Umfang fortpflanzt. Bei hartem Material müsste ein wesentlich größeres Drehmoment aufgebracht werden, um es an der Sollbruchstelle über den gesamten Umfang gleichzeitig aufreißen zu lassen.

Es ist bereits bekannt, den Brechstopfen mit einem äußeren Bund zu formen, der beim Eindrücken bzw. Einschlagen des Brechstopfens in den Schlauch an dessen Ende zur Anlage kommt, also einen Anschlag bildet. Erfindungsgemäß ist nunmehr vorgesehen, dass ein solcher am unteren Teil des Brechstopfens angeformter, ringförmiger Bund stirn- und/oder umfangsseitig durch Kleben oder Schweißen materialschlüssig mit dem oberen Bereich der Verschlusskappe dicht verbunden wird. Auf diese Weise erhält man eine definierte Verbindungsfläche. Wenn am Ende des unteren Teils des Brechstopfens in üblicher Weise ein Außengewinde angeformt ist, bietet der ringförmige Bund die Möglichkeit, eine Verschluss kappe einzusetzen, deren Innendurchmesser größer ist als der Außendurchmesser des Außengewindes auf dem Brechstopfen. Man braucht dann kein Innengewinde mehr in der Verschlusskappe, so dass deren Formwerkzeug und der Formvorgang wesentlich vereinfacht werden können.

Um die Verschlusskappe mit Bezug auf den Brechstopfen, bei dem sich üblicherweise der untere Bereich des inneren Hohlraums zur Öffnung hin konisch erweitert (Luerkonus), zu zentrieren, empfiehlt sich eine Ausführung, bei der die Verschlusskappe mit einem passenden, konischen oder zylindrischen Zapfen in den konischen Teil des Hohlraums eingreift. Damit wird auch sichergestellt, dass die Sollbruchstelle in der Umfangswand der Verschlusskappe nicht durch eine radiale Relativbewegung aufgebrochen werden kann.

Ausführungsbeispiele der Erfindung werden nachstehend anhand der Zeichnung näher erläutert. Es zeigen:
- Fig. 1: einen Längsschnitt durch einen Brechverschluss mit einer mit der unteren Stirnfläche eines an einem Brechstopfen angeformten Bundes durch Kleben oder Schweißen materialschlüssig dicht verbundenen Verschlusskappe;
- Fig. 2: einen Längsschnitt durch einen Brechverschluss, bei dem die Verschlusskappe rechts und links der Mittellinie mit unterschiedlicher Gestaltung hauptsächlich auf der Umfangsfläche des Bundes durch Kleben oder Schweißen materialschlüssig ringsum dicht befestigt ist, und
- Fig. 3 + 4: Längsschnitte durch weitere Ausführungsbeispiele für die Verbindung zwischen dem Bund und der Verschlusskappe.

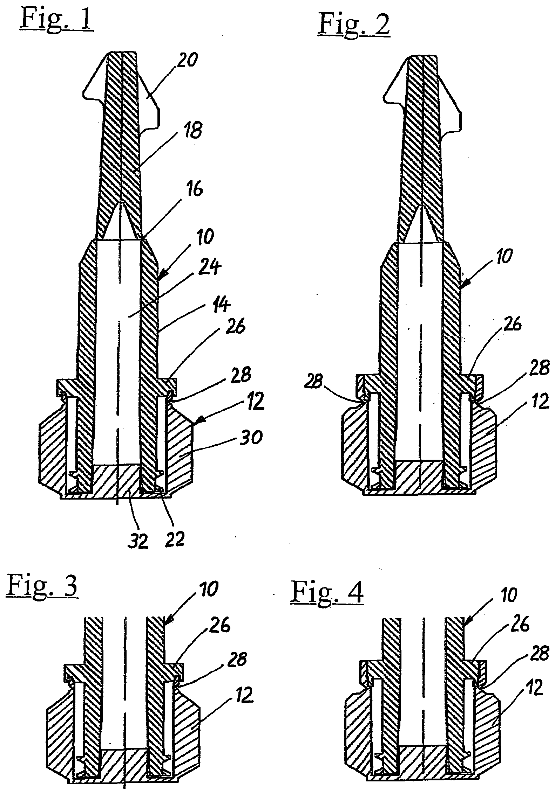

Der Brechverschluss nach Fig. 1 besteht aus einem Brechstopfen 10 und einer Verschlusskappe 12. Der Brechstopfen 10 gliedert sich in einen hohlen unteren Teil 14, dessen Außendurchmesser stramm sitzend in einen Auslassschlauch z. B. eines mit einer Natriumchloridlösung gefüllten Beutels passt. Über eine bei 16 gezeigte Sollbruchstelle schließt sich an den unteren Teil 14 ein im Querschnitt kleinerer oberer Teil 18 an, der an seinem oberen Ende mit radial abstehenden Nasen 20 geformt ist. Sie greifen innen an der Schlauchwand an und sorgen dafür, dass nach dem Abbrechen des oberen Teils 18 vom unteren Teil 14 und einer axialen Relativverschiebung der beiden Teile die an der Sollbruchstelle 16 gebildete Einlassöffnung geöffnet bleibt.

In weiterer Übereinstimmung mit der DE 41 116 474 C2 ist das untere Ende des Brechstopfens 10 mit einem Außengewinde 22 und einer konischen Erweiterung des inneren Hohlraums 24 geformt. Abweichend von dieser bekannten Ausführung ist allerdings, dass die Verschlusskappe 12 nicht mit einem Innengewinde in das Außengewinde 22 eingreift, sondern mit einem zylindrischen inneren Hohlraum geformt ist, dessen Durchmesser größer ist als der Außendurchmesser des Außengewindes 22. Die Verschlusskappe 12 kann daher durch eine einfache axiale Bewegung von unten auf den unteren Bereich des Brechstopfens 10 aufgeschoben werden, um dann mit ihrem obersten Endbereich an einem ringförmigen Bund 26 auf dem äußeren Umfang des unteren Teils 14 des Brechstopfens 10 festgeklebt oder angeschweißt zu werden. Im Ausführungsbeispiel nach Fig. 1 ist die Unterseite des Bundes 26 mit einer abgeschrägten Stufe geformt und das obere Ende der Verschluss kappe 12 mit einer entsprechenden, sich nach oben konisch erweiternden Öffnung. Die beiden Teile 10, 12 sind an der gezeigten Konusfläche und/oder an den aneinander anliegenden Stirnflächen an der Unterseite des Bundes 26 und dem oberen Ende der Verschlusskappe 12 miteinander materialschlüssig verbunden.

Nur ein wenig unterhalb ihrer Befestigungsfläche ist die Verschlusskappe 12 mit einer bei 28 gezeigten Sollbruchstelle geformt. Im übrigen hat sie auf der äußeren Umfangsfläche axiale Rippen 30 und auf ihrem geschlossenen Boden einen in den konischen Teil des inneren Hohlraums 26 des Brechstopfens 10 eingreifenden konischen oder zylindrischen Zapfen 32, der im Zusammenwirken mit der inneren Umfangswand des Hohlraums 24 für eine Zentrierung der Teile 10, 12 sorgt.

Der Brechstopfen 10 sitzt von vornherein im Auslassschlauch des angelieferten, gefüllten Beutels, wobei das freie Ende des Auslassschlauchs an der oberen Stirnfläche des Bundes 26 anliegt. Wenn die medizinische Lösung gebraucht wird, dreht man zunächst an der Verschluss kappe 12, während man durch den Auslassschlauch hindurch den Brechstopfen 10 an seinem unteren Teil 14 oberhalb des Bundes 26 festhält. Bei der Drehbewegung der Verschluss kappe 12 reißt die Sollbruchstelle 28 auf, und die Verschlusskappe 12 wird abgenommen. Anschließend wird ein Überleitungsgerät mit einem zum Außengewinde 22 und zum Luerkonus am unteren Ende des inneren Hohlraums 24 passenden Anschlussstück an den Brechstopfen 10 angekuppelt. Nachdem diese Leitungsverbindung hergestellt worden ist, wird durch eine durch den Auslassschlauch hindurch wirksame Biegebelastung von Hand der obere Teil 18 vorn unteren Teil 14 des Brechstopfens abgebrochen und dabei innerhalb des Auslassschlauchs nach oben verschoben, so dass die medizinische Lösung an dem im Querschnitt kleineren oberen Teil 18 des Brechstopfens 10 vorbei in die durch das Zerbrechen an der Sollbruchstelle 16 gebildete Öffnung in den unteren Teil 14 des Brechstopfens 10 eintreten und durch den inneren Hohlraum 24 hindurch in das Überleitungsgerät zum Patienten hin strömen kann.

Das Ausführungsbeispiel nach Fig. 2 unterscheidet sich nur dadurch von dem nach Fig. 1, dass das rechts und links der Mittellinie unterschiedlich gestaltete obere Ende der Verschlusskappe 12 hauptsächlich mit einer stufenförmig abgesetzten zylindrischen Innenfläche auf der zylindrischen Außenfläche des Bundes 26 durch Kleben oder Schweißen materialschlüssig mit dem Brechstopfen 10 verbunden ist. Auch hier wieder befindet sich mit geringem Abstand axial unterhalb der Verbindungsfläche eine Sollbruchstelle 28 in der Umfangswand der Verschlusskappe 12.

Die Ausführungsbeispiele nach Fig. 3 und 4 zeigen weitere Möglichkeiten der materialschlüssigen Verbindung zwischen der Verschlusskappe 12 und dem Bund 26. Gemäß Fig. 3 ist dieser stirnseitig mit einer Ringnut geformt, in welche das freie Ende der Verschlusskappe eingreift. Vorzugsweise ist die Ringnut mit wenigstens einer konischen Wandfläche ausgebildet, an die sich eine entsprechend konisch geformte Fläche am oberen Ende der Verschlusskappe anlegt.

Bei der Ausführung nach Fig. 4 ist der Bund 26 mit einem zum unteren Ende des Brechstopfens 10 weisenden Kragen geformt, so dass eine verlängerte Anlagefläche auf der Umfangsseite des Bundes 26 erhalten wird. Wie auch bei den anderen Ausführungsbeispielen sind zusammenwirkende stirnseitige Anschlagflächen vorgesehen, die beim Zusammenfügen und Verbinden die axiale Endstellung der Verschlusskappe 12 definieren. In diesem Fall kommt die Verschlusskappe an der unteren stirnseitigen Fläche des Kragens am Bund 26 zur Anlage.

Statt der materialschlüssigen Verbindung an einem Bund 26 könnte in dessen axialem Bereich auch ein Außengewinde auf dem Brechstopfen vorhanden sein, das mit einem passenden Innengewinde in der Verschlusskappe 12 in Eingriff zu bringen und anschließend materialschlüssig zu verbinden ist.

Es versteht sich, dass der vorgeschlagene Brechverschluss weitgehend unabhängig ist von der Formgebung des Brechstopfens und der Verschlusskappe im Einzelfall, sofern die für die Erfindung wichtigen Funktionen gewährleistet sind.

## Patentansprüche

1. Brechverschluss zum Verstopfen eines an einen Beutel oder Behälter mit einer medizinischen Lösung angeschlossenen Schlauchs bis zur Ingebrauchnahme, bestehend aus einem Brechstopfen (10) mit einem im Querschnitt entsprechend dem inneren Schlauchquerschnitt bemessenen, hohlen, endseitig offenen, unteren Teil (14), an dessen Ende ein Außengewinde (22) angeformt ist und wobei das untere Teil (14) einen angeformten Bund (26) aufweist, und einen über eine Sollbruchstelle (16) einstückig mit dem unteren Teil (14) verbundenen, im Querschnitt kleineren, oberen Teil (18), wobei die Öffnung des unteren Teils (14) durch eine dessen Ende übergreifende, lösbare Verschlusskappe (12) abgedeckt ist, die eine Sollbruchstelle (28) in der Umfangswand aufweist, **dadurch gekennzeichnet, dass** der Innendurchmesser der Verschlusskappe (12) größer ist als der Außendurchmesser des Außengewindes (22) des Brechstopfens (10) und die Verschlusskappe (12) in ihrem oberen Bereich mit dem angeformten Bund (26) stirn- und/oder umfangsseitig durch Kleben oder Schweißen materialschlüssig, ringsum dicht verbunden und die Sollbruchstelle (28) in der Umfangswand axial darunter ausgebildet ist.

2. Brechverschluss nach Anspruch 1, **dadurch gekennzeichnet, dass** sich der untere Bereich des inneren Hohlraums (24) des Brechstopfens (10) zur Öffnung hin konisch erweitert und die Verschlusskappe (12) mit einem passenden, konischen oder zylindrischen Zapfen (32) in den konischen Teil des Hohlraums (24) eingreift.

3. Brechverschluss nach einem der Ansprüche 1 oder 2, **dadurch gekennzeichnet, dass** die Verschlusskappe (12) aus einem thermoplastischen Elastomer (TPE) mit einer Härte von vorzugsweise maximal 50 Shore besteht.

## Claims

1. A breakable connection for sealing a tube connected to a bag or container with a medicinal solution until use, consisting of a breakable stopper (10) with a hollow lower part (14) open at its end and corresponding in cross section to the inner tube cross section, and on the end of the lower part (14) an outer thread (22) is formed, whereby the lower part (14) comprises a collar (26) formed thereupon, and an upper part (18) with a smaller cross-section, connected in one piece with the lower part (14) via a break point (16), whereby the opening of the lower part (14) is covered by a removable sealing cap (12) extended over the end of the lower part (14), whereby the sealing cap (12) comprises a break point (28) in the circumferential wall, **characterized in that** the inner diameter of the sealing cap (12) is greater than the outer diameter of the outer thread (22) of the breakable stopper (10) and in its upper region the sealing cap (12) is materially, sealingly connected all around a front and/or circumferential side with the collar (26) through adhering or welding and the break point (28) is formed axially thereunder in the circumferential wall.

2. A breakable stopper according to claim 1, **characterized in that** the lower region of the inner hollow space (24) of the breakable stopper (10) expands conically towards the opening and the sealing cap (12) extends into the conical part of the hollow space (24) with a matching, conical or cylindrical stud (32).

3. A breakable stopper according to one of the claims 1 or 2, **characterised in that** the sealing cap (12) is made of a thermoplastic elastomer (TPE) with a hardness of preferably a maximum of 50 Shore.

## Revendications

1. Fermeture à rupture destinée à boucher un tuyau raccordé à une poche ou un récipient contenant une solution médicinale jusqu'à son utilisation, constituée d'un bouchon à rupture (10) avec une partie inférieure (14), ouverte côté extrémité, creuse et dimensionnée en section transversale de manière correspondante à la section transversale intérieure du tuyau, partie inférieure sur l'extrémité de laquelle un filetage extérieur (22) est formé, la partie inférieure (14) comportant un collet (26) formé dessus, et avec une partie supérieure (18), plus petite en section transversale et reliée d'un seul tenant à la partie inférieure (14) par l'intermédiaire d'un point destiné à la rupture (16), l'ouverture de la partie inférieure (14) étant couverte par un capuchon (12) amovible, qui passe par-dessus l'extrémité de ladite partie inférieure et qui comporte un point destiné à la rupture (28) dans sa paroi périphérique, **caractérisée en ce que** le diamètre intérieur du capuchon (12) est plus grand que le diamètre extérieur du filetage extérieur (22) du bouchon à rupture (10), **en ce que** le capuchon (12) dans sa zone supérieure est assemblé de manière étanche et tout autour au collet (26), du côté frontal et/ou circonférentiel, par liaison de matière par collage ou soudage, et **en ce que** le point destiné à la rupture (28) dans la paroi périphérique est conçu axialement en dessous.

2. Fermeture à rupture selon la revendication 1, **caractérisée en ce que** la zone inférieure de la cavité intérieure (24) du bouchon à rupture (10) est élargie en cône en direction de l'ouverture et **en ce que** le capuchon (12) pénètre avec un bout (32) adapté, conique ou cylindrique, dans la partie conique de la cavité (24).

3. Fermeture à rupture selon l'une des revendications 1 ou 2, **caractérisée en ce que** le capuchon (12) est en un élastomère thermoplastique (TPE) ayant une dureté de 50 Shore au maximum de préférence.
